# FASCICULE DE BREVET EUROPEEN

(11) **EP 1 689 763 B1**
(45) Date de publication et mention de la délivrance du brevet: **28.05.2008**
(21) Numéro de dépôt: 04805342.5
(22) Date de dépôt: 29.10.2004
(51) Int. Cl.: C07H 1/00, C07H 3/02, C07H 3/04

(54) **PROCEDE DE PREPARATION DE SUCRES CETOSES PAR ISOMERISATION DE SUCRES ALDOSES**
VERFAHREN ZUR HERSTELLUNG VON KETOSEZUCKER, DAS DIE ISOMERISIERUNG VON ALDOSEZUCKERN BEINHALTET
KETOSE SUGAR PREPARATION METHOD COMPRISING ISOMERISATION OF ALDOSE SUGARS

(30) Priorité: 02.12.2003 FR 0314133
(43) Date de publication de la demande: 16.08.2006
(73) Titulaire: Flamma, 92330 Sceaux (FR)
(72) Inventeur: CORNILLE, Fabrice, F-91440 Bures sur Yvette (FR)
(74) Mandataire: Keib, Gérard
(86) Numéro de dépôt international: PCT/FR2004/002788
(87) Numéro de publication internationale: WO 2005/066192

(56) Documents cités:
- US-A- 3 256 270
- US-A- 3 822 249

## Description

La présente invention décrit un procédé industriel de production de sucres cétoses tels que le tagatose, le fructose et le lactulose par isomérisation de sucres aldoses tels que le galactose, le glucose et le lactose respectivement.

Les cétohexoses, aussi appelés cétoses, peuvent être utilisés comme agent édulcorant à faible teneur calorique (Tagatose) ou couramment utilisés pour le traitement de certaines affections intestinales (Lactulose).

L'isomérisation des aldoses par action de bases aqueuses telles que Ca(OH)2, NaOH, KOH est connue depuis longtemps.

Cette réaction d'isomérisation est connue sous le nom de « Conversion de Lobry de Bruyn-Van Ekenstein » d'après le nom de ses inventeurs (Rec. Trav. Chim. Pays-Bas 1895 (14) 203 and 1896 (15) 92).

Ces procédés présentent les inconvénients de dégrader les sucres de façon aléatoire, donnant ainsi de faibles rendements et nécessitant des étapes complexes d'extraction afin d'éliminer les produits de dégradation.

Une autre famille de procédé utilise l'aluminate de sodium et le tétraborate de sodium (voir brevets US-3,256,270; US-3,822,249 ; US-4,957,564, EP-375046).

Ces procédés, bien que permettant d'obtenir des rendements plus élevés et des produits plus purs, ne sont pas acceptables industriellement à cause de la difficulté présentée par la filtration de l'hydroxyde d'aluminium et par l'élimination totale de l'acide borique.

Un procédé amélioré est décrit dans le brevet US-6,214,124 dans lequel l'isomérisation du lactulose est obtenue par une catalyse à l'aluminate de sodium suivie par une étape de neutralisation effectuée par traitement au dioxyde de carbone gazeux sous pression dans un réacteur de type « Buss ». Cependant, la séparation du solide, constitué essentiellement d'hydroxyde d'aluminium et de bicarbonate de sodium, est difficile et requiert un filtre presse.

Afin de pallier à ces inconvénients, il était utile de mettre au point un procédé simple et économique de séparation de l'aluminium des solutions de sucres une fois l'isomérisation accomplie.

Le but de la présente invention est ainsi de remédier aux inconvénients des procédés connus.

Suivant l'invention le procédé de préparation de sucres cétoses est caractérisé en ce qu'il consiste à mettre une solution ou une suspension dans l'eau de sucre de type aldose en réaction avec de l'aluminate de potassium afin de convertir l'aldose en cétose par une réaction d'isomérisation, le mélange ainsi obtenu étant ensuite acidifié par traitement avec de l'acide sulfurique, pour obtenir un précipité de sulfate de potassium et d'aluminium que l'on sépare du milieu réactionnel par filtration.

Il a été constaté que ce procédé d'isomérisation d'aldose en cétose permet une filtration facile des sels d'aluminium, grâce au fait que la conversion de l'aldose en cétose est effectuée en utilisant l'aluminate de potassium comme catalyseur.

Après isomérisation des sucres aldoses catalysée par l'aluminate de potassium, l'aluminium est précipité sous forme de cristaux de sulfate d'aluminium et de potassium dodécahydrate par une étape d'acidification du milieu réactionnel à l'acide sulfurique.

Ce sel d'aluminium possède une faible solubilité dans les solutions aqueuses, contrairement au sel de sodium correspondant (voir Merck index 12^{th} édition, page 377 et 378).

En conséquence, ce sel est facilement séparable des solutions isomérisées par filtration.

Selon une version préférée de l'invention, le procédé comprend les étapes suivantes :
a. on prépare une solution ou une suspension aqueuse d'aldose,
b. on ajoute à cette solution ou suspension de l'aluminate de potassium,
c. on maintient le milieu en réaction pendant une durée suffisante pour obtenir une conversion quasi-totale de l'aldose en cétose,
d. on acidifie le mélange obtenu avec de l'acide sulfurique pour obtenir un pH compris entre 2 et 3,5,
e. on sépare par filtration le précipité de sulfate de potassium et d'aluminium.

De préférence, également, l'aldose est choisi parmi le galactose, le glucose et le lactose.

Avantageusement, la solution ou suspension aqueuse initiale d'aldose renferme 25 à 60% en poids d'aldose.

La proportion molaire d'aluminate de potassium ajoutée à la solution ou suspension aqueuse d'aldose est comprise entre 0,25 et 1 et de préférence égale à 0,4 par rapport à l'aldose.

Bien que le procédé puisse être utilisé sur un large éventail de température et de durée, l'étape c. de conversion de l'aldose en cétose est réalisée à une température comprise entre 15 et 70° C pendant 0,45 h à 20 h et de préférence entre 50 - 55° C pendant 2 h à 4 h.

Néanmoins, le procédé est cependant flexible puisque le rendement maximal peut être obtenu en faisant varier la durée de la réaction à une température donnée dans les intervalles de temps et de température autorisée.

Une fois la réaction terminée, le pH du milieu réactionnel est abaissé de 12,5 - 13 à pH 2 - 3,5 à l'aide d'acide sulfurique. A ce nouveau pH, l'aluminium précipite sous la forme de cristaux de sulfate d'aluminium et de potassium dodécahydrate qui, grâce à sa faible solubilité, est facilement séparable du milieu réactionnel par filtration. Le filtrat est ensuite débarrassé des produits de dégradation et des sels résiduels par passage sur résine échangeuse d'ions, par exemple du type Amberlite IR 120 et IRA 96.

La solution finale est enfin concentrée sous pression réduite à une température inférieure à 50° C.

Le sulfate d'aluminium et de potassium dodécahydrate récupéré à partir du milieu réactionnel peut être, selon des techniques connues de l'homme de l'art, suspendu dans de l'eau et mélangé avec la quantité nécessaire de KOH pour former à nouveau de l'aluminate de potassium réutilisable ou recyclable pour une nouvelle réaction de conversion d'aldose en cétose.

Afin d'illustrer le procédé correspondant à la présente invention, les exemples suivants sont rapportés.

### Exemple 1 :

100 g de Galactose est mis en suspension dans 100 ml d'eau. La suspension obtenue est chauffée sous agitation à 35° C. Une solution composée de 58 g d'aluminate de potassium K₂Al₂O₄.3H₂O dans 40 ml d'eau est ajoutée au milieu réactionnel. La suspension se transforme alors en une solution claire. La température est maintenue à 55° C pendant 3 heures. Le mélange réactionnel est ensuite refroidi à 0 - 5° C. A la solution réfrigérée est ensuite ajoutée une solution d'acide sulfurique à 30% (environ 300 g) sous constante et vigoureuse agitation, tandis que la température du milieu réactionnel augmente à 35° C. Le pH de la solution est ainsi ajusté entre 2 et 3,5. Le mélange réactionnel est refroidi à nouveau à 10° C sous lente agitation afin que le sulfate de potassium et d'aluminium dodécahydrate précipite. Ce précipité est éliminé facilement par filtration. Le filtrat est ensuite déionisé et purifié par passage sur colonnes de résines échangeuses d'ions suivant des procédures connues (résines Amberlite IR 120 et IRA 96).

La solution obtenue est ensuite concentrée sous pression réduite à une température de 45° C. La solution finale est analysée par HPLC et présente une composition de 83,9% de Tagatose, 12,6% de Galactose, 3,4% de Sorbose basée sur l'extrait sec.

### Exemple 2 :

100 g de Glucose et 58 g d'aluminate de potassium sont solubilisés dans 100 ml d'eau suivant la procédure décrite dans l'exemple 1.

L'analyse HPLC montre que la solution finale présente une composition de 85,3% de fructose, 11,3% de glucose basée sur l'extrait sec.

### Exemple 3 :

100 g de Lactose monohydrate et 29 g d'aluminate de potassium sont solubilisés dans 100 ml d'eau suivant la procédure décrite dans l'exemple 1.

L'analyse HPLC montre que la solution finale présente une composition de 85,0% Lactulose, 8,4% de Lactose, 5,2% Galactose et 1,4% Tagatose basée sur l'extrait sec.

## Revendications

1. Procédé de préparation de sucres cétoses dans lequel une solution ou une suspension dans l'eau de sucre de type aldose est mise en réaction avec de l'aluminate de potassium afin de convertir l'aldose en cétose par une réaction d'isomérisation, le mélange ainsi obtenu est acidifié par traitement avec de l'acide sulfurique, pour obtenir un précipité de sulfate de potassium et d'aluminium que l'on sépare du milieu réactionnel par filtration.

2. Procédé selon la revendication 1, **caractérisé en ce qu'**il comprend les étapes suivantes :
a. on prépare une solution ou une suspension aqueuse d'aldose,
b. on ajoute à cette solution ou suspension de l'aluminate de potassium,
c. on maintient le milieu en réaction pendant une durée suffisante pour obtenir une conversion quasi-totale de l'aldose en cétose,
d. on acidifie le mélange obtenu avec de l'acide sulfurique pour obtenir un pH compris entre 2 et 3,5,
e. on sépare par filtration le précipité de sulfate de potassium et d'aluminium.

3. Procédé selon l'une des revendications 1 ou 2, **caractérisé en ce que** l'aldose est choisi parmi le galactose, le glucose et le lactose.

4. Procédé selon l'une des revendications 1 à 3, **caractérisé en ce que** la solution ou suspension aqueuse initiale d'aldose renferme 25 à 60% en poids d'aldose.

5. Procédé selon l'une des revendications 1 à 4, **caractérisé en ce que** la proportion molaire d'aluminate de potassium ajoutée à la solution ou suspension aqueuse d'aldose est comprise entre 0,25 et 1 et de préférence égale à 0,4, par rapport à l'aldose.

6. Procédé selon l'une des revendications 2 à 5, **caractérisé en ce que** l'étape c. de conversion de l'aldose en cétose est réalisée à une température comprise entre 15 et 70° C pendant 0,45 h à 20 h et de préférence entre 50 - 55° C pendant 2 h à 4 h.

7. Procédé selon l'une des revendications 2 à 6, **caractérisé en ce que** le filtrat obtenu à l'étape e. est désalée par passage sur des résines échangeuses d'ions.

8. Procédé selon l'une des revendications 2 à 7, **caractérisé en ce que** le précipité de sulfate de potassium et d'aluminium obtenu à l'étape e. est traité avec de l'hydroxyde de potassium.

9. Procédé selon la revendication 8, **caractérisé en ce que** l'aluminate de potassium obtenu est recyclé dans l'étape b.

## Claims

1. Process for the preparation of ketose sugars, in which a solution or a suspension in water of aldose-type sugar is reacted with potassium aluminate in order to convert the aldose into ketose by an isomerization reaction, the mixture thus obtained is acidified by treatment with sulphuric acid, in order to obtain a precipitate of aluminium and potassium sulphate which is separated from the reaction medium by filtration.

2. Process according to claim 1, **characterized in that** it comprises the following steps:
a. a solution or an aqueous suspension of aldose is prepared,
b. potassium aluminate is added to this solution or suspension,
c. the reaction medium is maintained for a sufficient period of time to obtain an almost total conversion of the aldose to ketose,
d. the mixture obtained is acidified with sulphuric acid in order to obtain a pH comprised between 2 and 3.5,
e. the aluminium and potassium sulphate precipitate is separated by filtration.

3. Process according to one of claims 1 or 2, **characterized in that** the aldose is chosen from galactose, glucose and lactose.

4. Process according to one of claims 1 to 3, **characterized in that** the initial aqueous suspension or solution of aldose comprises 25 to 60% by weight of aldose.

5. Process according to any one of claims 1 to 4, **characterized in that** the molar proportion of potassium aluminate added to the aqueous suspension or solution of aldose is comprised between 0.25 and 1 and preferably equal to 0.4, with respect to the aldose.

6. Process according to one of claims 2 to 5, **characterized in that** step c. of conversion of the aldose to ketose is carried out at a temperature comprised between 15 and 70 °C for 0.45 h to 20 h and preferably between 50-55 °C for 2 h to 4 h.

7. Process according to one of claims 2 to 6, **characterized in that** the filtrate obtained in step e. is desalinated by passing over ion exchange resins.

8. Process according to one of claims 2 to 7, **characterized in that** the precipitate of aluminium and potassium sulphate obtained in step e. is treated with potassium hydroxide.

9. Process according to claim 8, **characterized in that** the potassium aluminate obtained is recycled in step b.

## Patentansprüche

1. Verfahren zur Herstellung von Ketosezucker, bei dem eine Lösung oder eine Suspension von Zucker vom Typ Aldose in Wasser mit Kaliumaluminat zur Reaktion gebracht wird, um die Aldose durch eine Isomerisationsreaktion in Ketose zu überführen, die auf diese Weise erhaltene Mischung durch Behandlung mit Schwefelsäure angesäuert wird, um einen Niederschlag von Kalium- und Aluminiumsulfat zu erhalten, den man vom Reaktionsmedium durch Filtration abtrennt.

2. Verfahren nach Anspruch 1, **dadurch gekennzeichnet, dass** es die folgenden Schritte umfasst:
a. man stellt eine wässrige Aldoselösung oder -suspension her,
b. man setzt dieser Lösung oder Suspension Kaliumaluminat zu,
c. man hält das Medium während einer Dauer in Reaktion, die ausreicht, um eine fast vollständige Überführung von Aldose in Ketose zu erhalten,
d. man säuert die erhaltene Mischung mit Schwefelsäure an, um einen pH-Wert zwischen 2 und 3,5 zu erhalten,
e. man trennt den Niederschlag von Kalium- und Aluminiumsulfat durch Filtration ab.

3. Verfahren nach einem der Ansprüche 1 oder 2, **dadurch gekennzeichnet, dass** die Aldose aus Galactose, Glucose und Lactose ausgewählt ist.

4. Verfahren nach einem der Ansprüche 1 bis 3, **dadurch gekennzeichnet, dass** die ursprüngliche wässrige Aldoselösung oder -suspension 25 bis 60 Gew.-% Aldose enthält.

5. Verfahren nach einem der Ansprüche 1 bis 4, **dadurch gekennzeichnet, dass** der molare Anteil von der wässrigen Aldoselösung oder -suspension zugesetztem Kaliumaluminat zwischen 0,25 und 1 beträgt und vorzugsweise gleich 0,4 ist, bezogen auf die Aldose.

6. Verfahren nach einem der Ansprüche 2 bis 5, **dadurch gekennzeichnet, dass** der Schritt c. der Überführung von Aldose in Ketose bei einer Temperatur zwischen 15 und 70° C während 0,45 h bis 20 h und vorzugsweise zwischen 50-55° C während 2 h bis 4 h durchgeführt wird.

7. Verfahren nach einem der Ansprüche 2 bis 6, **dadurch gekennzeichnet, dass** das im Schritt e. erhaltene Filtrat durch Passage über Ionenaustauschharze entsalzt wird.

8. Verfahren nach einem der Ansprüche 2 bis 7, **dadurch gekennzeichnet, dass** der im Schritt e. erhaltene Kalium- und Aluminiumsulfatniederschlag mit Kaliumhydroxid behandelt wird.

9. Verfahren nach Anspruch 8, **dadurch gekennzeichnet, dass** das erhaltene Kaliumaluminat in den Schritt b. rezykliert wird.
